(19) Europäisches Patentamt
European Patent Office
Office européen des brevets

(11) Publication number: 0 346 108
A2

(12) # EUROPEAN PATENT APPLICATION

(21) Application number: 89305774.5

(22) Date of filing: 07.06.89

(51) Int. Cl.$^4$: C 07 H 19/09
C 07 H 19/073, A 61 K 31/70

(30) Priority: 09.06.88 GB 8813680
21.02.89 GB 8903927

(43) Date of publication of application:
13.12.89 Bulletin 89/50

(84) Designated Contracting States:
AT BE CH DE ES FR GB GR IT LI LU NL SE

(71) Applicant: THE WELLCOME FOUNDATION LIMITED
183-193 Euston Road
London NW1 2BP (GB)

(72) Inventor: Rahim, Saad George
The Welcome Research Lab. Langley Court
Beckenham Kent (GB)

(74) Representative: Garrett, Michael et al
The Wellcome Research Laboratories Group Patents and Agreements Langley Court
Beckenham Kent BR3 3BS (GB)

(54) **Anti-infective nucleosides.**

(57) The present invention relates to mono-, di- and tri- esters and ethers of 5-alkynyl-substituted pyrimidine nucleosides and their use in medical therapy particularly in the treatment and prophylaxis of herpes viral infections. Also provided are pharmaceutical formulations and processes for the preparation of compounds according to the invention.

EP 0 346 108 A2

**Description**

## ANTI-INFECTIVE NUCLEOSIDES

The present invention relates to esters of 5-alkynyl pyrimidine nucleosides and their use in medical therapy particularly for the treatment or prophylaxis of certain herpes virus infections.

Of the DNA viruses, the herpes group is the source of the most common viral illnesses in man. The group consists of herpes simplex virus (HSV), varicella zoster virus (VZV), cytomegalovirus (CMV) and Epstein-Barr virus (EBV).

Varicella zoster virus (VZV) is a herpesvirus which causes chicken-pox and shingles. Chicken-pox is the primary disease produced in a host without immunity and in young children is usually mild illness characterised by a vesicular rash and fever. Shingles or zoster is the recurrent form of the disease which occurs in adults who were previously infected with varicella-zoster virus. The clinical manifestions of shingles are characterised by neuralgia and a vesicular skin rash that is unilateral and dermatomal in distribution. Spread of inflammation may lead to paralysis or convulsions. Coma can occur if the meninges become affected. In immunodeficient patients VZV may disseminate causing serious or even fatal illness. VZV is of serious concern in patients receiving immunosuppressive drugs for transplant purposes or for treatment of malignant neoplasia and is a serious complication of AIDS patients due to their impaired immune system.

In common with other herpes viruses, infection with CMV leads to a lifelong association of virus and host and, following a primary infection, virus may be shed for a number of years. Clinical effects range from death and gross disease (microcephaly, hepatosplenomegaly, jaundice, mental retardation) through failure to thrive, susceptibility to chest and ear infections to a lack of any obvious ill effect. CMV infection in AIDS patients is a predominant cause of morbidity as; in 80% of the adult population, it is present in a latent form and can be re-activated in immuno-compromised patients.

Epstein-Barr virus (EBV) causes infectious mononucleosis, and is also suggested as the causative agent of nasopharyngeal cancer, immunoblastic lymphoma, Burkitt's lymphoma and hairy leukoplakia.

European Patent Publication No. 272065 describes and claims the synthesis and use of 5-alkynyl pyrimidine nucleosides and their pharmaceutically acceptable derivatives in medical therapy, particularly in the treatment or prophylaxis of human viral infections such as herpes infections.

We have now surprisingly discovered that certain esters of 5-prop-1-ynyl- and 5-ethynyl- substituted pyrimidine nucleosides are of particular value in medical therapy especially for the treatment of certain viral infections as described below.

The compounds referred to above may be represented by the following general formula (I):

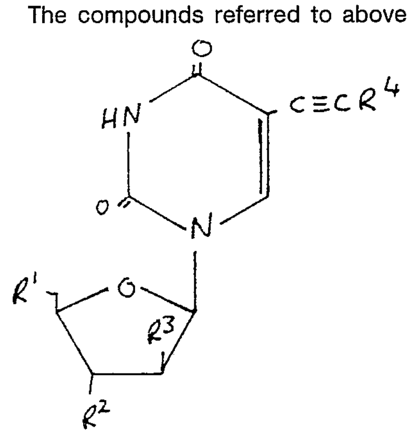

wherein $R^1$ represents a hydroxy group, an amino acid ester group or a carboxylic acid ester group in which the non-carbonyl moiety of the ester grouping is selected from straight or branched chain $C_{1-6}$ hydroxyalkyl or $C_{1-6}$ alkyl (e.g. propyl, butyl, isopropyl, isobutyl, isovaleryl), $C_{3-7}$ cycloalkyl (e.g. cyclohexyl), heterocycloalkyl (e.g. 4-morpholino), heterocycloalkenyl (e.g. 2-furyl), $C_{1-6}$ alkoxy (e.g. ethoxy), $C_{1-6}$ alkoxyalkyl (e.g. methoxymethyl), $C_{1-6}$ carboxyalkyl (e.g. carboxyethyl), $C_{1-6}$ aminoalkyl (e g. N.N-diethylaminoethyl), carbamoylalkyl (e.g. N,N-diethylcarbamoyl propionyl), aralkyl (e.g. benzyl), aryloxyalkyl (e.g. phenoxymethyl), aryl (e.g. phenyl) optionally substituted by halogen, $C_{1-6}$ alkyl or $C_{1-6}$ alkoxy, or a mono-, di- or tri-phosphate ester, or an ether group; $R^2$ represents a hydroxy group, an amino acid or a carboxylic acid ester group as defined for $R^1$, a monophosphate ester, or an ether group; $R^3$ represents a hydrogen atom, a hydroxy group, or an ester group, or an ether group as defined for $R^1$; $R^4$ represents a hydrogen atom or a methyl group; or a pharmaceutically acceptable salt thereof provided that at least one of $R^1$, $R^2$ and $R^3$ represents an ester or an ether group and provided that when $R^4$ is hydrogen, $R^3$ is not hydrogen or $R^1$, $R^2$ and $R^3$ do not all represent acetyl groups; or provided that when $R^4$ is a methyl group and $R^3$ is hydrogen, $R^1$ and $R^2$ do not both represent toluoyl groups or do not both represent acetyl groups; or provided that when $R^4$ is methyl, $R^1$, $R^2$ and $R^3$ do not all represent toluoyl groups or do not all represent acetyl groups or when $R^2$ and $R^3$ represent hydroxy groups, $R^1$ does not represent a morpholinocarbonyl, dimethylcarbonyl, carboxypropionyl, t-butoxycarbonyl group or when $R^1$ and $R^2$ represent hydroxy groups, $R^3$ does not represent a

dimethylaminocarbonyl group.

Preferred compounds of formula (1) are those wherein $R^4$ represents a methyl group. Also preferred are those compounds of formula (1) wherein $R^2$ and $R^3$ represent hydroxy groups and $R^1$ represents an ester as defined above most preferably a carboxylic acid ester.

Preferred esters are those wherein the non-carbonyl moiety of the carboxylic acid ester grouping is selected from straight or branched chain $C_{1-6}$ alkyl, $C_{1-6}$ alkoxy or $C_{3-7}$ cycloalkyl.

Also desirable are compounds wherein the ester group is an amino acid ester group for example an L-valyl group.

Particularly preferred compounds are those wherein $R^4$ represents a methyl group, $R^2$ and $R^3$ represent hydroxy groups and $R^1$ represents a carboxylic acid ester in which the non-carbonyl moiety of the ester grouping is branched $C_{1-6}$ alkyl, namely propyl or isopropyl.

Also preferred are the compounds wherein $R^4$ represents a methyl group, $R^1$ and $R^3$ represent hydroxy groups and $R^2$ represents a carboxylic acid ester in which the non-carbonyl moiety of the ester grouping is branched $C_{1-6}$ alkyl namely isobutyl.

Where reference is made to a $C_{1-6}$ alkyl moiety this includes methyl, ethyl, propyl, butyl, pentyl and hexyl.

Most preferred compounds are:-

1. 1-(5-0-Isobutyryl-β-D-arabinofuranosyl)-5-prop-1-ynyluracil;
2. 1-(5-0-Cyclohexylcarbonyl-β-D-arabinofuranosyl)-5-prop-1-ynyluracil;
3. 1-(5-0-(3-Methylbutyryl)-β-D-arabinofuranosyl)-5-prop-1-ynyluracil;
4. 1-(5-0-Ethoxycarbonyl-β-D-arabinofuranosyl)-5-prop-1-ynyluracil;
5. 5-Prop-1-ynyl-1-(3-0-trimethylacetyl-β-D-arabinofuranosyl)uracil;
6. 1-(5-0-Butyryl-β-D-arabinofuranosyl)-5-prop-1-ynyluracil;
7. 1-(5-0-(2-Ethylbutyryl)-β-D-arabinofuranosyl)-5-prop-1-ynyluracil.
8. 5-Prop-1-ynyl-1-(5-0-L-valyl-β-D-arabinofuranosyl)uracil

Hereinafter the compounds mentioned above will be referred to as compounds according to the invention.

In tests in rats measuring the urinary recovery as the parent compound, (% dose administered) after oral administration, the compounds according to the invention show a large increase in absorption from the gut compared with the parent compound and compared with certain other esters of the parent compound. This enables less drug to be adminstered while still providing equivalent drug levels in the plasma after oral absorption. Furthermore the compounds according to the invention retain the antiviral activity of the parent compound, thus there is no loss of antiviral effectivity associated with the advantageous increase in bioavailability.

The present invention further includes:

a) Compounds according to the invention for use in the treatment or prophylaxis of viral infections particularly herpes virus infections selected from VZV, CMV and EBV infections;

b) A method for the treatment or prophylaxis of a viral infection in a human selected from VZV, CMV and EBV infections which comprises treating a person identified as having an infection with an effective amount of a compound according to the invention.

c) Use of a compound according to the invention in the manufacture of a medicament for the treatment or prophylaxis of a herpes virus infection selected from VZV, CMV and EBV infections.

It should be noted that compounds according to the invention may be particularly useful for the treatment of VZV infections.

Examples of the clinical conditions caused by such herpes viruses as CMV, VZV and EBV infections which may be treated in accordance with the invention include those referred to above.

Salts according to the invention which may be conveniently used in therapy include physiologically acceptable base salts, eg. derived from an appropriate base, such as alkali metal (e.g. sodium), alkaline earth metal (e.g. magnesium) salts, ammonium and $NX_4^+$ (wherein X is $C_{1-4}$ alkyl) salts. Alternatively salts may be prepared by reaction of the esterified compound with a base such as sodium hydride to form the corresponding sodium salt.

The compounds according to the invention may be administered by any route appropriate to the condition to be treated, suitable routes including oral, rectal, nasal, topical (including buccal and sublingual), vaginal and parenteral (including subcutaneous, intramuscular, intravenous, intradermal, intrathecal and epidural). It will be appreciated that the preferred route may vary with, for example, the condition of the recipient.

For each of the above-indicated utilities and indications the amount required of the individual active ingredient will depend upon a number of factors including the severity of the condition to be treated and the identity of the recipient and will ultimately be at the discretion of the attendant physician. In general, however, for each of these utilities and indications, a suitable, effective dose will be in the range 0.1 to 250 mg per kilogram body weight of recipient per day, preferably in the range 1 to 100 mg per kilogram body weight per day and most preferably in the range 5 to 30 mg per kilogram body weight per day; an optimum dose is about 15 mg per kilogram body weight per day (unless otherwise indicated all weights of active ingredient are calculated as the parent compound; for salts and esters thereof the figures would be increased proportionately.) The dose may if desired be presented as two, three, four or more sub-doses administered at appropriate intervals throughout the day. These sub-doses may be administered in unit dosage forms, for example, containing 10 to 1000 mg, preferably 20 to 500 mg and most preferably 100 to 400 mg of active ingredient per unit dosage form.

The compounds of the present invention may be administered alone or in combination with other

therapeutic agents, for example, with 9-(2-hydroxyethoxymethyl)guanine (acyclovir) used to treat herpes virus infections in particular HSV (1); with zidovudine used to treat retroviral infections in particular HIV infections; with other antiviral nucleosides, or any other agents which when in combination with a compound according to the invention provide a beneficial therapeutic effect.

While it is possible for the compounds to be administered alone it is preferable to present them as pharmaceutical formulations. The formulations of the present invention comprise at least one active ingredient, as above defined, together with one or more acceptable carriers thereof and optionally other therapeutic ingredients. The carrier(s) must be "acceptable" in the sense of being compatible with the other ingredients of the formulation and not deleterious to the recipients thereof.

The formulations include those suitable for oral, rectal, nasal, topical (including buccal and sublingual), vaginal or parenteral (including subcutaneous, intramuscular, intravenous, intradermal, intrathecal and epidural) administration. The formations may conveniently be presented in unit dosage form and may be prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, or paste or may be contained within liposomes.

A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g. povidone, gelatin, hydroxypropylmethyl cellulose), lubricant, inert diluent, disintegrant (e.g. sodium starch glycollate, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethyl-cellulose in varying proportions to provide the desired release profile.

A capsule may be made by filling a loose or compressed powder on an appropriate filling machine, optionally with one or more additives. Examples of suitable additives include binders such as povidone, gelatin, lubricants, inert diluents, disintegrants as for tablets. Capsules may also be formulated to contain pellets or discrete sub-units to provide slow or controlled release of the outline ingredient. This can be achieved by extruding and spheronising a wet mixture of the drug plus an extrusion acid (e.g. microcrystalline cellulose) plus a diluent such as lactose. The spheroids thus produced can be coated with a semi-permeable membrane (e.g. ethyl cellulose, Eudragit WE30D) to produce sustained release properties.

For infections of the eye or other external tissues, e.g., mouth and skin, the formulations are preferably applied as a topical ointment or cream containing the active ingredient in an amount of, for example, 0.075 to 20% w/w, preferably 0.2 to 15% w/w and most preferably 0.5 to 10% w/w. When formulated in an ointment, the active ingredients may be employed with either a paraffinic or a water-miscible ointment base. Alternatively, the active ingredients may be formulated in a cream with an oil-in-water cream base or as in a water in oil base.

If desired, the aqueous phase of the cream base may include, for example, at least 40-45% w/w of a polyhydric alcohol, i.e. an alcohol having two or more hydroxyl groups such as propylene glycol, butane-1,3-diol, mannitol, sorbitol, glycerol and polyethylene glycol and mixtures thereof. The topical formulations may desirably include a compound which enhances absorption or penetration of the active ingredient through the skin or other affected areas. Examples of such dermal penetration enhancers include dimethylsulphoxide and related analogues.

The oily phase of the emulsions of this invention may be constituted from known ingredients in a known manner. While this phase may comprise merely an emulsifier (otherwise known as an emulgent), it desirably comprises a mixture of at least one emulsifier with a fat or an oil or with both a fat and an oil. Preferably, a hydrophilic emulsifier is included together with a lipophilic emulsifier which acts as a stabilizer. It is also preferred to include both an oil and a fat. Together, the emulsifer(s) with or without stabilizer(s) make up the so-called emulsifying wax, and the wax together with the oil and/or fat make up the so-called emulsifying ointment base which forms the oily dispersed phase of the cream formulations.

Emulgents and emulsion stabilizers suitable for use in the formulation of the present invention include Tween 60, Span 80, cetostearyl alcohol, myristyl alcohol, glyceryl mono-stearate and sodium lauryl sulphate.

The choice of suitable oils or fats for the formulation is based on achieving the desired cosmetic properties, since the solubility of the active compound in most oils likely to be used in pharmaceutical emulsion formulations is very low. Thus the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of

branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used.

Formulations suitable for topical administration to the eye also include eye drops wherein the active ingredient is dissolved or suspended in a suitable carrier, especially an aqueous solvent for the active ingredient. The active ingredient is preferably present in such formulations in a concentration of 0.5 to 20%, advantageously 0.5 to 10% particularly about 1.5% w/w.

Formulations suitable for topical administration in the mouth include lozenges comprising the active ingredient in a flavoured basis, usually sucrose and acacia or tragacanth; pastilles comprising the active ingredient in an inert basis such as gelatin and glycerin, or sucrose and acacia; and mouth-washes comprising the active ingredient in a suitable liquid carrier.

Formulations for rectal administration may be presented as a suppository with a suitable base comprising for example cocoa butter or higher fatty alcohol (e.g. hard wax European Pharmacopoeia) or triglycerides and saturated fatty acids (e.g. Witepsol).

Formulations suitable for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as for example a nasal spray or as nasal drops, include aqueous or oily solutions of the active ingredient.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose, as herein above recited, or an appropriate fraction thereof, of an active ingredient.

It should be understood that in addition to the ingredients particularly mentioned above the formulations of this invention may include other agents conventional in the art having regard to the type of formulation in question, for example those suitable for oral administration may include flavoring agents.

The present invention also provides a process for the preparation of a compound according to the invention which comprises:-

A. reacting a compound of formula (II)

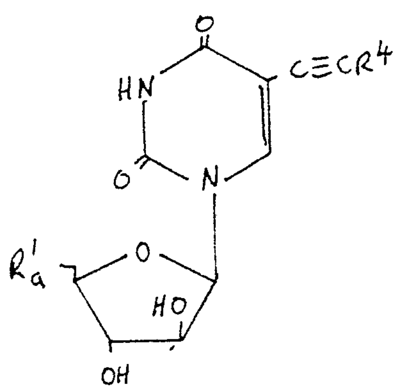

(II)

wherein $R^4$ is as hereinbefore defined, and $R^1_a$ is a hydroxy group or an appropriate hydroxy-protecting group with a compound serving to provide the appropriate ester grouping(s) at the 2,3 and/or 5-position of the sugar; or

B. reacting a compound of formula (III)

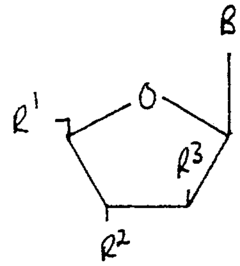

(III)

wherein $R^1$, $R^2$ and $R^3$ are as hereinbefore defined and B is a purine or pyrimidine base, with a 5-alkynyl uracil base; or

C. reacting a compound of formula (IV)

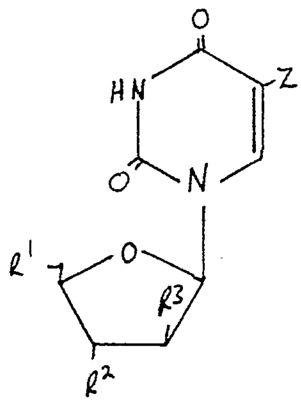

(IV)

wherein Z is a leaving group and $R^1$, $R^2$ and $R^3$ are as hereinbefore defined with a compound serving to provide the appropriate alkynyl group, and

optionally thereafter or simultaneously therewith, performing either or both of the following, in any desired order:

i) removing any protecting groups;

ii) where the resulting compound is a compound of formula (I), converting it into a pharmaceutically acceptable salt thereof or, where the resulting compound is a pharmaceutically acceptable salt, converting it into a different pharmaceutically acceptable salt or a compound of formula (I).

With regard to process A, the hydroxy-protecting groups may be trityl or silyl protecting groups for example 4-methoxytrityl. The compounds of formula (I) may be prepared from corresponding compounds of formula (II) the preparation of which is described in EP Publication No. 272065 for example by esterification using an appropriate acid chloride or anhydride, advantageously in the presence of a base such as pyridine or triethylamine which may also serve as a solvent medium for the reaction at a temperature in the range of 0°-70°C advantageously 0°-30°C. The ratio of acylating agent to compound of formula (II) is preferably 1.2:1 for monoesterification of the 5-position of the sugar moiety. Deprotection of the 5-position of the sugar is carried out by acid treatment with acetic acid at a temperature greater than 50°C.

Alternatively, the compound of formula (I) may be prepared from the corresponding compound of formula (II) by transesterification using an appropriate ester of the corresponding acid (e.g. methyl) in the presence of a base such as pyridine or triethylamine which may also serve as a solvent medium for the reaction.

In addition, the esterification reaction may be carried out for example in a solvent such as pyridine or dimethylformamide and in the case when an acid is used, in the presence of a coupling agent such as N,N′-dicyclohexylcarbodiimide, optionally in the presence of a catalytic base such as 4-dimethylaminopyridine. The water formed during the reaction may, if desired, be removed in conventional manner, for example by distillation or by the addition of a water-binding substance. Subsequently, the ester obtained as reaction product may be isolated in conventional manner.

With regard to process B, group B is preferably a purine or pyrimidine base capable of donating the esterified sugar to a 5-alkynyluracil base using for example an enzyme such as a phosphorylase enzyme in the presence of a phosphate salt at a pH of 5.0 - 9.0 and a temperature of 15° - 90°C advantageously 40 - 60°C.

With regard to process C, the compound of formula (IV) may be prepared by esterification of a suitable halogenated nucleoside such as 1-(β-D-arabinofuranosyl)-5-iodouracil with any of the ester groups referred to above according to process A. The compounds of formula (I) may be prepared from the corresponding compounds of formula (IV) by reaction with an appropriate acetylene, such as propyne, or protected acetylene, such as trimethylsilylacetylene, with a palladium catalyst and a copper (1) salt catalyst in the presence of an organic base, such as triethylamine, at an elevated temperature such as 50°C followed by selective removal of protecting groups as necessary (as exemplified by Robins, M.J. and Barr, P.J. in J. Org. Chem. (1983), 48,1854 et seq.). A preferred palladium catalyst is bis(triphenylphosphine) palladium dichloride

and a preferred copper catalyst is cuprous iodide.

Salts according to the invention may be prepared in conventional manner for example by reaction of the parent compound with an appropriate base to form the corresponding base salt. Other derivatives according to the invention can also be prepared in conventional manner.

The following examples illustrate the present invention:-

Example 1

1-(5-0-Isobutyryl-β-D-arabinofuranosyl)-5-prop-1-ynyluracil

To a stirred solution of 1-(β-D-arabinofuranosyl)-5-prop-1-ynyluracil (prepared according to EP Publication 272065, 1g, 3.5mmol) in dry pyridine (10ml) at 0°C under $N_2$ was added dropwise, a solution of isobutyryl chloride (0.4ml, 3.85mmol) in dry dichloromethane (10ml) over a period of 20 minutes. Stirring was continued at 0°C for 2 hours then at room temperature for 2 hours. A further quantity of isobutyrylchloride (0.05ml) was added and stirring maintained for a further 1 hour. The solvent was evaporated under reduced pressure, and residual pyridine co-evaporated with portions of ethanol (3x50ml) to give a white foam. Chromatographic separation in silica gel column eluting with 8% $MeOH/CH_2Cl_2$ gave pure product which was triturated with ether to give a white solid.

Yield: 0.744g (60%)

Mpt: 195-197°C

| | | | |
|---|---|---|---|
| Analysis Calculated: | C-54.59, | H-5.682, | N-7.95% |
| Found: | C-54.12, | H-5.684, | N-7.786% |

δ($d_6$DMSO) 11.55(1H,bs,NH), 7.6(1H,s,H-6), 6.03(1H,d,H-1′), 5.74 (1H,d,OH-2′), 5.61(1H,m,OH-3′),4.4-4.15(2H,m,H-5′), 4.1-3.85(3H,m,H-2′,H-3′,H-4′), 2.6(1H,h,$(CH_3)_2\underline{CH}$), 1.98(3H,s,C≡$C\underline{CH_3}$), 1.15(3H,d,$(\underline{CH_3})_2CH$); 1.13ppm(3H,d,$(\underline{CH_3})_2CH$).

Example 2

1-(5-0-Cyclohexylcarbonyl-β-D-arabinofuranosyl)-5-prop-1-ynyluracil

To a stirred solution of 1-(β-D-arabinofuranosyl)-5-prop-1-ynyluracil (prepared according to EP Publication 272065, 0.28g, 1mmol) in dry pyridine (5ml) at 0°C under dry $N_2$ was added, dropwise, a solution of cyclohexane carboxylic acid chloride (0.16ml, 0.18g, 1.2mmol) in dry dichloromethane (5ml) over a period of 10 minutes. The mixture was stirred at 0°C for 90 minutes then at room temperature for 90 minutes. The solvent was evaporated under reduced pressure and residual pyridine co-evaporated with ethanol to give an oil. Chromatographic separation on a silica gel column eluting with 8% $MeOH/CH_2Cl_2$ gave pure product which was triturated with ether to give a white solid.

Yield: 0.17g (46%)

Mpt: 167-168°C

| | | | |
|---|---|---|---|
| Analysis Calculated: | C-50.59, | H-5.08, | N-7.86% |
| Found : | C-50.55, | H-5.15, | N-7.79% |

δ($d_6$DMSO) 11.55(1H,bs,NH), 7.58(1H,s,H-6), 6.O(1H,d,H-1′), 5.71(1H,d,OH-2′), 5.6(1H,m,OH-3′), 4.4-4.15(2H,m,H-5′), 4.05-3.85(3H,m,H-2′ ,H-3′,H-4′), 2.45-2.27(1H,m,$\underline{CH}C=O$), 1.96(3H,s,C≡$C\underline{CH_3}$), 1.9-1.1 ppm (10H,m,cyclohexyl H's)

Example 3

1-(5-0-Isovaleryl-β-D-arabinofuranosyl)-5-prop-1-ynyluracil

To a stirred solution of 1-(β-D-arabinofuranosyl)-5-prop-1-ynyluracil (prepared according to EP Publication 272065, 0.28g, 1mmol) in dry pyridine (5ml) at 0° C under dry $N_2$ was added, dropwise, a solution of iso-valeryl chloride (0.15ml, 0.14g, 1.2mmol) in dry dichloromethane (5ml) over a period of 10 minutes. The mixture was stirred at 0° C for 90 minutes and the solvent was removed by evaporation under reduced pressure. Residual pyridine was co-evaporated with portions of ethanol (3x25ml) to give an oil which was purified on a flash silica column eluting with 6% $MeOH/CH_2Cl_2$ to give pure product. Trituration with ether gave a white solid.

Yield: 0.080g (22%)

Mpt: 132-135° C

| | | | |
|---|---|---|---|
| Analysis Calculated: | C-55.74, | H-6.011, | N-7.65% |
| Found: | C-55.90, | H-5.723, | N-7.522% |

δ($d_6$DMSO) 11.55(1H,bs,NH), 7.61(1H,s,H-6), 6.0(1H,d,H-1′), 5.71(1H,d,OH-2′), 5.62(1H,m,OH-3′), 4.4-4.15(2H,m,H-5′), 4.07-3.85(3H,m,H-2′,H-3′,H-4′), 2.25(2H,d,$\underline{CH_2}C=O$), 2.15-1.9(1H,m,$(CH_3)_2\underline{CH}$),

1.97(3H,s,C≡CHCH3), 0.93 ppm (6H,d,(CH3)2CH).ra2|

Example 4

1-(5-0-Ethoxycarbonyl-β-D-arabinofuranosyl)-5-prop-1-ynyluracil

To a stirred solution of 1-(β-D-arabinofuranosyl)-5-prop-1-ynyluracil (prepared according to EP Publication 272065, 0.3g, 1.06mmol) in dry pyridine (5ml) at 0°C under dry $N_2$ was added dropwise over 5 minutes ethylchloroformate (0.122ml, 1.27mmol). The mixture was stirred at room temperature for 5 hours and the solvent evaporated under reduced pressure. Residual pyridine was co-evaporated with portions of ethanol and the final residue purified by column chromatography eluting with 10% $MeOH/CH_2Cl_2$ to give the product, isolated following trituration with ether.

Yield: 0.17g (46%)

Mpt: 167-168°C

| | | | |
|---|---|---|---|
| Analysis Calculated: | C-50.59, | H-5.08, | N-7.86% |
| Found: | C-50.55, | H-5.15, | N-7.79% |

Example 5

1-(5-0-Benzoyl-β-D-arabinofuranosyl)-5-prop-1-ynyluracil

To a stirred solution of 1-(β-D-arabinofuranosyl)-5-prop-1-ynyluracil (prepared according to EP Publication 272065, 0.28,, 1mmol) in dry pryidine (5ml) at 0°C under dry $N_2$, was added dropwise over 10 minutes benzoyl chloride (0.14ml, 1.2mmol) in dichloromethane. The mixture was stirred at 0°C for 90 minutes and at room temperature overnight. The solvent was evaporated under reduced pressure and residual pyridine co-evaporated with portions of ethanol. The final residue was purified by column chromatography eluting with 6% $MeOH/CH_2Cl_2$ to give the product, isolated following trituration with ether.

Yield: 0.19g (50%)

Mpt: 210°C (dec)

| | | | |
|---|---|---|---|
| Analysis Calculated: | C-59.07, | H-4.66, | N-7.25% |
| Found: | C-59.21, | H-4.59, | N-6.98% |

Example 6

1-(5-0-(4-Methoxytrityl)-β-D-arabinofuranosyl)-5-prop-1-ynyluracil

1-(β-D-Arabinofuranosyl)-5-prop-1-ynyluracil (prepared according to EP Publication 272065 2g, 7.1mmol), 4-methoxytrityl chloride (2.79g, 9.6mmol) and crushed 4°A molecular sieves (20g) were combined in dry dichloromethane (40ml) and dry pyridine (8ml) and stirred at room temperature for 48 hours. The mixture was filtered, the solid washed with dichloromethane (2 x 30ml) and the combined filtrate and washings was evaporated to dryness. Purification of the residue by column chromatography eluting with 8% $MeOH/CH_2Cl_2$ afforded the title compound which was isolated following trituration with ether.

Yield: 2g (51.3%).

Example 7

1-(3-0-Acetyl-5-0-(4-methoxytrityl)-β-D-arabinofuranosyl)-5-prop-1-ynyluracil

To a stirred solution of the product of Example 6 (0.4g, 0.72mmol) in dry pyridine (5ml) at O°C was added slowly acetyl chloride (0.062g, 0.79mmol) and stirring maintained at 0°C for 30 minutes and room temperature for 2 hours. A further aliquot of acetyl chloride (0.027g,0.34mmol) was added at 0°C and after stirring at room temperature for a further 2 hours, the solvent was evaporated. Residual pyridine was co-evaporated with portions of ethanol and the final residue purified by column chromatography eluting with 5% $MeOH/CH_2Cl_2$ . Isolation of the two major products afforded the title compound (Yield=0.13g, 29%) and 1-(2,3-di-0-acetyl--5-0-(4-methoxytrityl)-β-D-arabinofuranosyl)-5-prop-1-ynyluracil. Yield:0.3 g (71%).

Example 8

1-(3-0-Acetyl-β-D-arabinofuranosyl)-5-prop-1-ynyluracil

A solution of 1-(3-0-acetyl-5-0-(4-methoxytrityl)-β-D-arabinofuranosyl) -5-prop-1-ynyluracil from Example 7 in 80% acetic acid and ethanol is heated at 90°C for 15 minutes and the solvent evaporated. Residual acetic acid is removed by coevaporation with portions of ethanol and the final residue triturated with ether to give a white solid which is filtered, washed with ether and dried and identified as the title compound.

Example 9

5-Prop-1-ynyl-1-(3-0-trimethylacetyl-β-D-arabinofuranosyl)uracil

To a stirred solution of the product of Example 6 (0.5g, 0.9mmol) in dry pyridine (5ml) at 0°C was added dropwise pivaloyl chloride (0.13ml, 1.08mmol) and the mixture was stirred at room temperature for 4 hours. A further aliquot of pivaloyl chloride (0.08ml, 0.66mmol) was added at 0°C and after stirring at room temperature for 2 hours the mixture was heated at 70°C for 2 hours and the solvent was evaporated. Residual pyridine was coevaporated with portions of ethanol and the crude product mixture was purified by column chromatography eluting with 10% $MeOH/CH_2Cl_2$. The two products isolated following trituration with ether were identified as the title compound (Yield=0.12g, 36%) and 1-(5-0-(4-methoxytrityl)-3-0-trimethylacetyl-β-D-arabinofuranosyl)-5-prop-1-ynyluracil, (Yield=0.1g, 17%).

Heating a solution of the 5-0-(4-methoxytrityl) intermediate in 80% acetic acid (4ml) and ethanol (2ml) at 90°C for one hour also afforded the title compound which was isolated by preparative layer chromatography eluting with 8% $MeOH/CH_2Cl_2$ to give a white solid.
Yield: 0.04g (72%).
Mpt: 108-9°C

Analysis for 0.22 hydrate.

| | | | |
|---|---|---|---|
| Calculated: | C-55.18, | H-6.06, | N-7.56% |
| Found: | C-55.4, | H-6.255, | N-7.125% |

δ($d_6$DMSO)7.81(1H,s,H-6), 5.95(2H,m,H-1′ and OH-5′), 5.7(1H,m,OH-2′), 4.95(1H,m,H-3′), 4.09(1H,m,H-2′), 3.89(1H,m,H-4′), 3.62(2H,m,H-5′), 1.98(3H,s,$C\equiv CCH_3$), 1.17 ppm (9H,s,t Bu).

Example 10

1-(3-0-Propionyl-β-D-arabinofuranosyl)-5-prop-1-ynyluracil

To a stirred solution of the product of Example 6 (0.5g, 0.9mmol) in dry pyridine (5ml) at 0°C is added dropwise, propionyl chloride (0.13ml, 1.08mmol) and the mixture stirred at room temperature for 4 hours and heated at 70°C for 2 hours and the solvent was evaporated. Residual pyridine is coevaporated with portions of ethanol and the crude product mixture purified by column chromatography eluting with 8% $MeOH/CH_2/Cl_2$. The product isolated following trituration with ether is identified as 1-(5-0-(4-methoxytrityl)-3-0-propionyl-β-D-arabinofuranosyl)-5-prop-1-ynyluracil.

Heating a solution of the 5-0-(4-methoxytrityl) intermediate in 80% acetic acid (4ml) and ethanol (2ml) at 90°C for one hour gives the title compound following chromatographic separation on silica gel eluting with 8% $MeOH/CH_2Cl_2$.

Example 11

1-(5-0-Methoxyacetyl-β-D-arabinofuranosyl)-5-prop-1-ynyluracil

To a solution of 1-(β-D-arabinofuranosyl)-5-prop-1-ynyluracil (prepared according to EP Publication 272065, 0.28g,1mmol) in dry pyridine (5ml) at 0°C under dry $N_2$ was added dropwise a solution of methoxyacetyl chloride (0.13g, 0.11ml, 1.2mmol) in dry dichloromethane (5ml) over a period of 10 minutes and the whole was stirred at 0°C for 2 hours. After stirring at room temperature for 3 hours, the solvent was evaporated under reduced pressure, residual pyridine coevaporated with several portions of ethanol (3x25ml) and the resulting oil was chromatographed on a silica gel column eluting with 8% $MeOH/CH_2Cl_2$. The appropriate fractions were combined, evaported to dryness and trituration with ether gave a white solid which was chromatographically pure.
Yield: 0.144g (41%)
Mpt: 176-178°C

| | | | |
|---|---|---|---|
| Analysis Calculated: | C-50.85, | H-5.085, | N-7-91% |
| Found: | C-50.72, | H-5.164, | N-7.91% |

Example 12

1-(5-0-Butyryl-β-D-arabinofuranosyl)-5-prop-1-ynyluracil 0.3 hydrate

To a solution of 1-(β-D-arabinofuranosyl)-5-prop-1-ynyluracil (prepared according to EP Publication 272065, 0.29g, 1.03mmol) in dry pyridine (5ml) at 0°C was added dropwise, a solution of butyryl chloride (0.13 ml, 1.2mmol) in dry dichloromethane (5ml) over a period of 10 minutes and the whole was stirred at 0°C for 1.5 hours. After stirring at room temperature for 2 hours the solvent was removed by evaporation under reduced pressure, residual pyridine co-evaporated with ethanol (3x25ml) and the resulting oil was chromatrographed

on a silica gel column eluting with 8% $MeOH/CH_2Cl_2$. The appropriate fractions were combined, evaporated to dryness and the residue was triturated with ether to give a white solid which was pure by tlc.
Yield: 0.113g (32%)
Mpt: 152-155°C

| | | | |
|---|---|---|---|
| Analysis Calculated: | C-53.72, | H-5.764, | N-7.83% |
| Found: | C-53.93, | H-5.62, | N-7.534% |

δ($d_6$DMSO) 11.56(1H,bs,NH), 7.63(1H,s,H-6), 6.04(1H,d,H-1′), 5.7(1H,d,OH-2′), 5.62(1H,m,OH-3′), 4.43-4.17(2H,m,H-5′), 4.1-3.87(3H,m,H-2′,H-3′,H-4′), 2.35(2H,t,$\underline{CH_2}C=O$, 1.99(3H,s,$C\equiv CCH_3$), 1.6(2H,sex, $CH_3\underline{CH_2}$), 0.9 ppm (3H,t,$\underline{CH_3}CH_2$.

Example 13

1-(5-0-tert-Butylacetyl-β-D-arabinofuranosyl)-5-prop-1-ynyluracil

To a solution of 1(-β-D-arabinofuranosyl)-5-prop-1-ynyluracil (prepared according to EP Publication 272065, 0.28g, 1mmol) in dry pyridine (5ml) at 0°C was added a solution of tert-butylacetyl chloride (0.17ml, 1.2mmol) in dry dichloromethane (5ml), dropwise over a period of 10 minutes. The mixture was left standing in the refrigerator for 2 days after which the solvent was evaporated under reduced pressure and residual pyridine co-evaporated with portions of ethanol (3x25ml). The resulting oil was chromatographed on a silica gel column eluting with 6% $MeOH/CH_2Cl_2$, the appropriate fractions were combined, evaporated to dryness and the residue was triturated with ether/petroleum ether to give a white solid.
Yield: 0.209% (55%)
Mpt: 185-188°C

| | | | |
|---|---|---|---|
| Analysis Calculated: | C-56.84, | H-6.316, | N-7.37% |
| Found: | C-56.62, | H-6.332, | N-7.201% |

Example 14

1-(5-0-Phenoxyacetyl-β-D-arabinofuranosyl-5-prop-1-ynyluracil

To a solution of 1-(β-D-arabinofuranosyl)-5-prop-1-ynyluracil (prepared according to EP Publication 272065, 0.28g, 1mmol) in dry pyridine (5ml) at 0°C was added dropwise, a solution of phenoxyacetyl chloride (0.17ml, 1.2mmol) in dry dichloromethane (5ml) over a period of 10 minutes and the whole was left standing in the refrigerator for 2 days. A further quantity of phenoxyacetyl chloride (0.05ml) was added and the mixture was stirred at room temperature overnight. The solvent was evaporated under reduced pressure, residual pyridine co-evaporated with portions of ethanol (3x25ml) and chromatographic separation of the residue on a silica gel column eluting with 8% $MeOH/CH_2Cl_2$ gave a slightly impure product. Recrystallisation from ethanol gave pure product as white crystals.
Yield: 0.142g (34%)
Mpt: 180-183°C

| | | | |
|---|---|---|---|
| Analysis Calculated: | C-57.69, | H-4.808, | N-6.73% |
| Found: | C-57.57, | H-4.824, | N-6.673% |

Example 15

1-(5-0-(2-Ethylbutyryl)-β-D-arabinofuranosyl)-5-prop-1-ynyluracil

To a solution of 1-(β-D-arabinofuranosyl)-5-prop-1-ynyluracil (prepared according to EP Publication 272065, 0.28g,1mmol) in dry pyridine (5ml) at 0°C under dry $N_2$ was added dropwise a solution of 2-ethylbutyryl chloride (0.17ml, 1.27mmol) in dry dichloromethane (5ml) over a period of 10 minutes and the whole was stirred at 0°C for 3 hours. A further aliquot of 2-ethylbutyryl chloride (0.05ml) was added and the reaction was maintained at room temperature for 3 hours. The solvent was evaporated under reduced pressure, residual pyridine coevaporated with several portions of ethanol (3x25ml) and the resulting oil was chromatographed on a silica gel column eluting with 10% $MeOH/CH_2Cl_2$. The appropriate fractions were combined, evaporated to dryness and recrystallised twice from ethanol to give a pure product.
Yield: 0.09g(27%)
Mpt: 177-178°C

| | | | |
|---|---|---|---|
| Analysis Calculated: | C-49.41, | H-4.706, | N-8.23% |
| Found: | C-49.18, | H-4.477, | N-8.007% |

δ($d_6$DMSO) 11.55 (1H,bs,NH), 7.62(1H,s,H-6), 6.02(1H,d,H-1′), 5.75(1H,m,OH-2′), 5.63(1H,m,OH-3′), 4.43-4.12(2H,m,H-5′), 4.1-3.85(3H,m,H-2′,H-3′,H-4′), 2.38-2.2(1H,m,$(CH_3CH_2)_2\underline{CH}$), 1.97(3H,s,$C{\equiv}CCH_3$), 1.66-1.4(4H,m,$(CH_3\underline{CH_2})_2C$), 0.95-0.79 ppm (6H,m, $(\underline{CH_3}CH_2)_2CH$).

Example 16

1-(5-0-Methoxycarbonyl-β-D-arabinofuranosyl)-5-prop-1-ynyluracil

To a stirred solution of 1-(β-D-arabinofuranosyl)-5-prop-1-ynyluracil (prepared according to EP Publication 272065, 0.28g, 1mmol) in dry pyridine (5ml) at 0°C was added dropwise a solution of methylchloroformate (0.09ml, 1.2mmol) in dry dichloromethane (5ml) over a period of 15 minutes and the whole was stirred at room temperature for 6 hours. A further aliquot of methylchloroformate (0.01 ml) was added and stirring continued at room temperature for 2 hours. The solvent was evaporated under reduced pressure, residual pyridine coevaporated with several portions of ethanol (3x25ml) and the resulting gum was purified by flash column chromatography eluting with 10% MeOH/$CH_2Cl_2$. The appropriate fractions were combined, evaporated to dryness and recrystallised twice from ethanol to give a pure product.

Yield: 0.09g (27%)

Mpt: 177-178°C

| | | | |
|---|---|---|---|
| Analysis Calculated: | C-49.41, | H-4.706, | N-8.23% |
| Found: | C-49.18, | H-4.477, | N-8.007% |

Example 17

1-(5-0-Phenylacetyl-β-D-arabinofuranosyl-)-5-prop-1-ynyluracil

To a stirred solution of 1-β-D-arabinofuranosyl-5-prop-1-ynyluracil (prepared according to EP Publication 272065, 0.28g, 1mmol) in dry pyridine (5ml) at 0°C was added dropwise over a period of 10 minutes a solution of phenylacetyl chloride (0.16ml, 1.2mmol) in dry dichloromethane (5ml) and the whole was stirred at 0°C for 4.5 hours. A further quantity of phenylacetyl chloride (0.05ml) was added and stirring was continued at 0°C for 4 hours. The solvent was evaporated under reduced pressure, residual pyridine co-evaporated with portions of ethanol (3x25ml) to give a yellow foam which was purified by flash column chromatography on silica gel eluting with 6% MeOH/$CH_2Cl_2$. The appropriate fractions were combined, evaporated to dryness and the resulting off-white solid was recrystallised from ethanol to give a white crystalline solid.

Yield: 0.113g (28%)

Mpt: 200-202°C

| | | | |
|---|---|---|---|
| Analysis Calculated: | C-60.00, | H-5.00, | N-7.00% |
| Found: | C-60.00, | H-5.026, | N-6.878% |

Example 18

1-(5-0-(2-Furoyl)-β-D-arabinofuranosyl)-5-prop-1-ynyluracil

To a stirred solution of 1-β-D-arabinofuranosyl-5-prop-1-ynyluracil (prepared according to EP Publication, 272065, 0.3g, 1.06mmol) in dry pyridine (5ml) at 0°C was added dropwise over a period of 10 minutes, a solution of 2-furoyl chloride (0.13ml, 1.28mmol) in dry dichloromethane (5ml) and the whole was stirred at 0°C for 1.5 hours. The solvent was removed by evaporation under reduced pressure, residual pyridine co-evaporated with portions of ethanol (3x25ml) and the resulting oil was chromatographed on a silica gel column eluting with 6% MeOH/$CH_2Cl_2$. The appropriate fractions were combined and evaporated to dryness and the residue was recrystallised from ethanol to give a white crystalline solid which was pure by tlc.

Yield: 0.175g (44%)

Mpt: 212-215°C

| | | | |
|---|---|---|---|
| Analysis Calculated: | C-54.25, | H-4.255, | N-7.45% |
| Found: | C-54.44, | H-4.265, | N-7.267% |

Example 19

1-(5-0-L-Alanyl-β-D-arabinofuranosyl)-5-prop-1-ynyluracil

To a stirred solution of N-fluorenylmethoxycarbonyl-L-alanine in dry dichloromethane at 0° C under nitrogen is added dropwise a solution of dicyclohexylcarbodiimide in dry dichloromethane and stirring maintained at 0° C for 5 minutes and at room temperature for 15 minutes. To the mixture is added a solution of 4-(N,N-dimethyl)aminopyridine and 1-(β-D-arabinofuranosyl)-5-prop-1-ynyluracil (prepared according to EP Publication, 272065) in dry N,N-dimethylformamide and stirring maintained for a further 1.0 hour. The reaction mixture is evaporated to dryness, dichloromethane added to the residue and the resulting white solid filtered off. The filtrate is evaporated to dryness and the residue purified by column chromatography eluting with methanol/chloroform (1:24) to give the Fmoc-protected ester.

The above product is treated with a solution of 20% dry piperidine/dimethylformamide and stirred at room temperature for 5 minutes. The mixture is evaporated to dryness under high vacuum and the residue washed with ether to give the title compound as a white solid.

Example 20

1-(5-0-(N-fluorenylmethoxycarbonyl-L-valyl)-β-D-arabinofuranosyl)-5-prop-1-ynyluracil

To a stirred solution of 1-(β-D-arabinofuranosyl)-5-prop-1-ynyluracil (prepared according to EP Publication 272065, 1g,3.54mmol), N-fluorenylmethoxycarbonyl-L-valine (1.32g, 3.89mmol) and N,N-dimethylaminopyridine (0.046g, 0.38mmol) in dry DMF (30ml) at 0° C was slowly added a solution of N,N′-dicyclohexylcarbodiimide (0.97g, 4.67mmol) in dry DMF (10ml) over 15 minutes. After the addition was complete, stirring was maintained at 5° C for 1.5 hours, 50% aqueous methanol (5ml) added and the mixture filtered. Evaporation of the filtrate under high vacuum with minimal heating and chromatographic separation of the residue on silica gel eluting with 10% $MeOH/CH_2Cl_2$ afforded the title compound as a crude product which was further purified by recrystallisation from acetone/toluene.

Yield: 0.196g (9%)

Mpt: 232° C

| | | | |
|---|---|---|---|
| Microanalysis: calculated | C-63.71, | H-5.47, | N-6.96% |
| Found: | C-64.2, | H-5.50, | 6.92% |

5-Prop-1-ynyl-1-(5-0-L-valyl-β-D-arabinofuranosyl)uracil

A solution of the product of Example 1 (0.17g, 0.28mmol) in 20% piperidine in dry DMF (2ml) was stirred at room temperature for 5 minutes and rapidly evaporated under high vaccum with minimal heating. The white residue was extracted with ether (4x10ml) to give the title compound.

Yield: 0.095g (89%)

Mpt: 161-162° C

| | | | |
|---|---|---|---|
| Microanalysis: calculated | C-52.51, | H-6.18, | N-10.81% |
| Found: | C-52.65, | H-6.19, | N-10.67% |

Example 21

1-(5-0-L-Isoleucinyl-β-Darabinofuranosyl)-5-proplynyluracil

To a stirred solution of N-fluorenylmethoxycarbonyl-L-isoleucine in dry dichloromethane at 0°C under nitrogen is added dropwise a solution of dicyclohexylcarbodiimide in dry dichloromethane and stirring maintained at 0° C for 5 minutes and at room temperature for 15 minutes. To the mixture is added a solution of 4-(N,N-dimethyl)aminopyridine and 1-(β-D-arabinofuranosyl)-5-prop-1-ynyluracil (prepared according to EP Publication No. 272065 in dry N,N-dimethylformamide and stirring maintained for a further 1.0 hour. The reaction mixture is evaporated to dryness, dichloromethane added to the residue and the resulting white solid filtered off. The filtrate is evaporated to dryness and the residue purified by column chromatography eluting with 4% $MeOH/CH_2Cl_2$ to give the Fmoc-protected ester.

The above product is treated with a solution of 20% dry piperidine/dimethylformamide and stirred at room temperature for 5 minutes. The mixture is evaporated to dryness under high vacuum and the residue washed with ether to give the title compound as a white solid.

Example 22

1-(5-0-(3-(N,N-Diethylcarbamoyl)propionyl-β-D-arabinofuranosyl)-5-prop-1-ynyluracil

To a stirred solution of N.N-diethylsuccinamic acid in dry dichloromethane at 0° C under nitrogen is added dropwise a solution of dicyclohexylcarbodiimide in dry dichloromethane and stirring maintained at 0° C for 5 minutes and at room temperature for 15 minutes. To the mixture is added a solution of 4-(N,N-dimethyl)aminopyridine and 1-(β-D-arabinofuranosyl)-5-prop-1-ynyluracil (present according to EP Publication, 272065) in dry N,N-dimethylformamide and stirring maintained for a further 1.0 hour. The reaction mixture is evaporated to dryness, dichloromethane added to the residue and the resulting white solid filtered off. The filtrate is evaporated to dryness and the residue purified by column chromatography eluting with 4% $MeOH/CH_2Cl_2$ to give the title compound.

Examples 23-25

23. 2′-Deoxy-3′,5′-di-O-acetyl-5-prop-1-ynyluridine

24. 5′-0-Acetyl-2′-deoxy-5-prop-1-ynyluridine

25. 3′-0-Acetyl-2′-deoxy-5-prop-1-ynyluridine

2′-Deoxy-5-prop-1-ynyluridine, (J. Med. Chem. 26(5) 661-666 [1983]) (5.33mg, 2mmol) was dissolved in dry pyridine (4ml) and acetic anhydride (204mg, 2mmol) was added. The mixture was left at room temperature for 24 hours. Pyridine was removed in vacuo at 40°C, and the residual oil was coevaporated with 2x5ml EtOH to a white solid. The product was separated into 3 fractions by flash chromotagraphy on silica gel eluting with 5% $MeOH/CH_2Cl_2$ to provide :

Example 23
Yield : 0.065g
Mpt : 155-156°C

| | | | |
|---|---|---|---|
| Analysis Calcd : | C-54.85, | H-5.18, | N-8.00% |
| Found : | C-54.92, | H-5.19, | N-7.71% |

Example 24
Yield : 0.187g
Mpt : 204-206°C

| | | | |
|---|---|---|---|
| Analysis Calcd : | C-54.54, | H-5.23, | N-9.09% |
| Found : | C-54.88, | H-5.22, | N-8.84% |

Example 25
Yield : 0.070g
Mpt : 175°C

| | | | |
|---|---|---|---|
| Analysis $0.5H_2O$ Calcd : | C-52.99, | H-5.40, | N-8.883% |
| Found : | C-52.82, | H-5.15, | N-8.87% |

Examples 26 and 27

26. 2′-Deoxy-5′-O-propionyl-5-prop-1-ynyluridine

27. 2′-Deoxy-3′-O-propionyl-5-prop-1-ynyluridine

The method of Example 23 was employed using propionic anhydride to introduce the ester group. Separation of the 3′- and 5′- esters was by flash chromatography.

Example 26
Mpt : 180-181°C

| | | | |
|---|---|---|---|
| Analysis Calcd : | C-55.89, | H-5.63, | N-8.69% |
| Found : | C-55.56, | H-5.51, | N-8.34% |

Example 27

Mpt : 174-175°C

| | | | |
|---|---|---|---|
| Analysis Calcd : | C-55.89, | H-5.63, | N-8.69% |
| Found : | C-55.90, | H-5.33, | N-8.67% |

Examples 28 and 29

28. 2′-Deoxy-5′-O-isobutyryl-5-prop-1-ynyluridine

29. 2′-Deoxy-3′-O-isobutyryl-5-prop-1-ynyluridine

The method of Example 23 was employed using isobutyric anhydride to introduce the ester group. Separation of the 3′- and 5′- esters was by flash chromatography.

Example 28

Mpt : 162-163°C

| | | | |
|---|---|---|---|
| Analysis Calcd : | C-57.13, | H-5.99, | N-8.33% |
| Found : | C-57.33, | H-5.78, | N-8.26% |

Example 29

Mpt : 179-180°C

| | | | |
|---|---|---|---|
| Analysis Calcd : | C-57.13, | H-5.99, | N-8.33% |
| Found : | C-56.85, | H-5.82, | N-8.29% |

Example 30

3′-0-Anisoyl-2′-deoxy-5-prop-1-ynyluridine

2′-Deoxy-5-prop-1-ynyluridine (J. Med. Chem. 26(5) 661-666 [1983]) (533mg, 2mmol) was dissolved in dry pyridine (5ml) and stirred at 0°C and 4-anisoyl chloride (341mg, 2mmol) was added. The mixture was stirred at room temperature for 5 hours. The pyridine was evaporated in vacuo at 40°C. The residue was coevaporated with 2x5ml EtOH to give a white glass. The product was purified by flash chromatography on silica gel eluting with 5% MeOH/$CH_2Cl_2$. Fractions containing only the 5-mono ester were pooled and evaporated to give 500mg of a white glass. The product was recrystallised from 30ml EtOH the white needles were filtered off and washed in $Et_2O$, then dried at 70°C under vacuum.

Yield : 0.380g (48%)

Mpt : 200-201°C

| | | | |
|---|---|---|---|
| Anal. Calc. | C-59.99, | H-5.04, | N-6.98% |
| Found | C-59.86, | H-5.01, | N-6.82% |

Example 31

2′-Deoxy-3′,5′-di-O-(4-anisoyl)-5-prop-1-ynyluridine

2′-Deoxy-5-propynyluridine (J. Med. Chem. 26(5) 661-666 [1983]) (533mg, 2mmol) was dissolved in dry pyridine (5ml) then stirred at 0°C and 4-anisoyl chloride (750mg, 4.4mmol) was added. The mixture was stoppered and stirred at room temperature overnight. The pyridine was removed at 40°C under high vacuum then the residue was coevaporated with 2x5ml EtOH to give a pale yellow solid. The solid was triturated well with EtOH, filtered off and washed with ether (0.99g). It was then taken up in 35ml of warm $CH_2Cl_2$ and diluted with 70ml EtOH and left at 0°C to give white crystals which were filtered off , washed with EtOH then $Et_2O$, and

dried at 70°C under vacuum.
Yield : 0.870g (81%)
Mpt : 214-215°C

| | | | |
|---|---|---|---|
| Anal. Calc. | C-62.91, | H-4.90, | N-5.24% |
| Found | C-62.63, | H-4.71, | N-4.99% |

Example 32

2′-Deoxy-3′ ,5′-di-O-(3-carboxypropionyl)-5-prop-1-ynyluridine

2′-Deoxy-5-propynyluridine (J. Med. Chem. 26(5) 661-666 [1983]) (1.066g, 4mmol) was dissolved in dry pyridine (8ml), dimethylaminopyridine (40mg) whilst succinic anhydride (0.92g, 9.2mmol) was added; the mixture was stirred until homogeneous, then left at room temperature for 2 days. The pyridine was removed at 40°C under high vacuum and the residue coevaporated with EtOH to remove the last traces. The residue was taken up in 100ml of warm water and 5ml EtOH, then cooled. White crystals were filtered off and dried in vacuo over $P_2O_5$.
Yield : 0.325g (17%)
Mpt : 127-130°C

| | | | |
|---|---|---|---|
| Anal. Calc. | C-51.50, | H-4.76, | N-6.01% |
| Found | C-51.64, | H-4.61, | N-5.81% |

Example 33

2′-Deoxy-3′ ,5′-di-O-acetyl-5-prop-1-ynyluridine

2′-Deoxy-5-propynyluridine (J. Med. Chem. 26(5) 661-666 [1983]) (533mg, 2mmol) was dissolved in dry pyridine (4ml) and acetic anhydride (0.414ml, 448mg, 4.4mmol) was added. The mixture was left at room temperature for 24 hours. The pyridine was removed under high vacuum at 40°C. The residue oil was coevaporated with 2x5ml EtOH to give a white solid which was recrystallised from 10ml EtOH. The white crystals were filtered off, washed in EtOH, $Et_2O$, then dried at 70°C under vacuum.
Yield : 0.520g (74%)
Mpt : 155-156°C

| | | | |
|---|---|---|---|
| Anal. Calc. | C-54.85, | H-5.18, | N-8.00% |
| Found | C-54.92, | H-5.19, | N-7.71% |

Example 34

2′-Deoxy-3′ ,5′-di-O-propionyl-5-prop-1-ynyluridine

The method of Example 33 was employed using propionic anhydride to introduce the ester groups.
Yield : 0.6g (79%)
Mpt : 149-150°C

| | | | |
|---|---|---|---|
| Anal. Calc. | C-57.13, | H-5.86, | N-7.40% |
| Found | C-57.20, | H-5.85, | N-7.33% |

Example 35

2′-Deoxy-3′ ,5′-di-O-isobutyryl-5-prop-1-ynyluridine

The method of Example 33 was employed using isobutyric anhydride to introduce the ester groups.
Yield : 0.7g (86%)
Mpt : 133-134°C

| | | | |
|---|---|---|---|
| Anal. Calc. | C-59.10, | H-6.45, | N-6.89% |
| Found | C-58.71, | H-6.25, | N-6.84% |

Example 36

2'-Deoxy-3' ,5'-di-O-pentanoyl-5-prop-1-ynyluridine

The method of Example 33 was employed using valeric anhydride to introduce the ester groups.

Yield : 0.4g (48%)

Mpt : 112-113°C

| | | | |
|---|---|---|---|
| Anal. Calc. | C-60.81, | H-6.96, | N-6.45% |
| Found | C-60.56, | H-7.03, | N-6.35% |

Example 37

5-Ethynyl-1-(2,3,5-tri-O-propionyl-β-D-arabinofuranosyl)uracil

1-(β-D-arabinofuranosyl)-5-ethynyluracil (J. Med. Chem. 26(5) 661-666 [1983]) (268mg 1mmol) and dry pyridine (3ml) were stirred at room temperature and propionic anhydride (0.427ml 430mg, 3.3mmol) were added. The mixture was stirred at room temperature for 60 hours. The pyridine was removed under high vacuum at 40°C and the residue coevaporated with 2x10ml EtOH to remove last traces of pyridine leaving a yellow oil. The product was purified by flash silica chromatography eluting with 3% $MeOH/CH_2Cl_2$. Fractions containing only major spot were pooled and evaporated to a white glass (340mg). The product was triturated with 60-80 petroleum ether and the solid filtered and dried under high vacuum.

Yield : 0.3g (69%)

Mpt : 58-64°C

| | | | |
|---|---|---|---|
| Anal. Calc. | C-52.86, | H-5.77, | N-6.17% |
| Found | C-52.79, | H-5.55, | N-5.91% |

Example 38

5-Ethynyl-1-(2,3,5-tri-O-isobutyryl-β-D-arabinofuranosyl)uracil

The method of Example 37 was employed using isobutyric anhydride to introduce the ester groups.

Mpt : 48-58°C

| | | | |
|---|---|---|---|
| Anal. Calc. | C-57.73, | H-6.32, | N-5.86% |
| Found | C-57.99, | H-6.55, | N-5.74% |

Example 39

5-Ethynyl-1-(2,3,5-tri-O-benzoyl-β-D-arabinofuranosyl)uracil

1-(β-D-arabinofuranosyl)-5-ethynyluracil (J. Med. Chem. 26(5) 661-666 [1983]) (268mg 1mmol) and dry pyridine (3ml) were stirred at room temperature and benzoyl chloride (0.366ml 443mg, 3.15mmol) were added. The mixture was stoppered and stirred at room temperature overnight. The pyridine was removed under high vacuum at 40°C and the residue was triturated with 15ml EtOH. The solid was filtered off washed with EtOH, then $Et_2O$ and dried. The product was taken up in a minimum volume of hot $CH_2Cl_2$, diluted with 2x volume of EtOH and then stood in the freezer. The white crystals were filtered off, washed in EtOH and $Et_2O$ to give the title compound.

Yield : 0.275g (47%)

Mpt : 227-228°C

| | | | |
|---|---|---|---|
| Anal. Calc. | C-66.20, | H-4.17, | N-4.83% |
| Found | C-66.50, | H-4.00, | N-4.75% |

Example 40

1-(5-O-Acetyl-β-D-arabinofuranosyl)-5-prop-1-ynyluracil

1-(β-D-Arabinofuranosyl)-5-prop-1-ynyluracil (J. Med. Chem. 26(5) 661-666 [1983]) (254mg, 1mmol) were dissolved in dry pyridine (2.0ml) and stirred in an ice-bath under dry $N_2$. A solution of acetyl chloride (0.08ml 86mg, 1.1mmol) in dry $CH_2Cl_2$ (2.0ml) was added slowly dropwise over 10 minutes. The mixture was stirred at 0°C for 1.5 hours. The solution was evaporated to dryness at 40°C under high vacuum. Traces of pyridine were removed by coevaporating the residue with 3x5ml of EtOH to provide a white solid residue which was purified by flash silica chromotagraphy eluting with 10% $MeOH/CH_2Cl_2$. The white solid was triturated with ether, filtered off, and dried at 70°C under vacuum to give the title compound.

Yield : 0.1g (34%)

Mpt : 177-179°C

| | | | |
|---|---|---|---|
| Anal. Calc. | C-51.85, | H-4.97, | N-8.64% |
| Found | C-51.65, | H-4.72, | N-8.47% |

Example 41

1-(5-0-Propionyl-β-D-arabinofuranosyl)-5-prop-1-ynyl uracil

1-(β-D-Arabinofuranosyl)-5-prop-1-ynyl uracil (J. Med. Chem. 26(5) 661-666 [1983]) (254mg, 1mmol) was dissolved in dry pyridine (2.0ml) and stirred at 0°C under dry $N_2$ and a solution of propionylchloride (0.096ml 102mg, 1.1mmol) in 2.0ml of dry $CH_2Cl_2$ was added slowly, dropwise, over 10 minutes. The mixture was stirred at 0°C for 1 1/2 hours then at room temperature for 1.5 hours. The solution was evaporated to dryness at 40°C under high vacuum. Traces of pyridine were removed by coevaporation with 3x5ml EtOH to provide a white solid which was purified by flash silica chromotagraphy eluting with 10% $MeOH/CH_2Cl_2$. The white solid product was triturated with ether, filtered off and dried at 70°C under vacuum to give the title compound.

Yield : 0.15g (49%)

Mpt : 165-167°C

| | | | |
|---|---|---|---|
| Anal. Calc. | C-53.25, | H-5.36, | N-8.28% |
| Found | C-52.98, | H-5.40, | N-8.11% |

Example 42

1-(5-0-Pentanoyl-β-D-arabinofuranosyl)-5-prop-1-ynyluracil

1-(β-D-Arabinofuranosyl)-5-prop-1-ynyluracil (J. Med. Chem 26(5) 661-666 [1983] (254mg, 1mmol) was dissolved in dry pyridine (2.0ml) and stirred at 0° C under dry $N_2$ and a solution of valeryl chloride (0.131ml 132.6mg, 1.1mmol) in 2.0ml of dry $CH_2Cl_2$ was added slowly, dropwise, over 10 minutes. The mixture was stirred at 0° C for 1.5 hours then at room temperature for 1.5 hours. The pyridine was removed at 40° C under high vaccum. The residue was coevaporated with 3x5ml ether. The solid product was purified by flash silica chromatrography eluting with 10% $MeOH/CH_2Cl_2$. The white solid product was triturated with ether, filtered off and dried at 70° C under vacuum to give the title compound.

Yield : 0.16g (49%)

Mpt. 154-156° C

| | | | |
|---|---|---|---|
| Anal. Calc. | C-55.73, | H-6.05, | N-7.65% |
| Found | C-55.39, | H-6.03, | N-7.55% |

Example 43

1-(5-0-(4-Anisoyl)-β-D-arabinofuranosyl)-5-prop-1-ynyluracil

1-β-D-Arabinofuranosyl-5-prop-1-ynyluracil (J. Med. Chem. 26(5) 661-666 [1983] (254mg, 1mmol) was dissolved in dry pyridine (2.0ml) and stirred at 0° C under dry $N_2$. A solution of anisoylchloride (200mg 1.2mmol) in 20ml dry dichloromethane was added dropwise over 5 minutes then stirred at 0°C for 1.5 hours then at room temperature for 1.5 hours. The pyridine was removed at 40°C under high vacuum. The residual oil was coevaporated with 3x5ml EtOH to provide a white solid which was washed with $CH_2Cl_2$ then dried at 70°C under vacuum to give the title compound.

Yield : 0.095g (26%)

Mpt : 220-222°C

| | | | |
|---|---|---|---|
| Anal. Calc. | C-57.69, | H-4.84, | N-6.73% |
| Found | C-57.44, | H-4.85, | N-6.75% |

Example 44

1-(2-O-Acetyl-β-D-arabinofuranosyl)-5-prop-1-ynyluracil

1-(β-D-Arabinofuranosyl)-5-prop-1-ynyluracil (J. Med. Chem. 26(5) 661-666 [1983]) (0.5g, 1.8mmol) was dissolved in dry dimethylformamide (5ml) imidazole (0.54g, 7.92mmol) was added and the solution was stirred at 0°C. Dichlorotetraisopropyldisiloxane (0.62ml 0.62g, 1.98mmol) was added and the mixture was stirred at room temperature for 3 hours. The solvent was evaporated to give an oil which was partitioned between $CH_2Cl_2$ and water. The organic layer was washed with $H_2O$, dried ($Na_2SO_4$) and evaporated to dryness to give a white foamy solid which was chromatographed on a silica gel column eluting with 4% $MeOH/CH_2Cl_2$. The resulting product (0.53g 1mmol) was dissolved in dry pyridine (5 ml) and to it was added acetic anhydride (0.1ml 1.1mmol) and the whole was stirred at room temperature for 3 hours. The solvent was evaporated under vacuum and the residue was coevaporated with ethanol and $CH_2Cl_2$ to give an intermediate which was taken up in tetrahydrofuran (5ml), tetrabutylammoniumfluoride tri-hydrate (0.63g 2mmol) was added and the mixture was stirred at room temperature for 30 minutes. The solvent was evaporated to dryness and the residue was chromatographed on silica gel eluting with 8% $MeOH/CH_2Cl_2$ to give the title compound.
Yield : 0.2g (51%)
Mpt : 172-175°C

| | | | |
|---|---|---|---|
| Anal. Calc. | C-51.85, | H-4.94, | N-8.64% |
| Found | C-51.89, | H-5.09, | N-8.13% |

Example 45

5-Prop-1-ynyl-1-(2,3,5-tri-O-isobutyryl-β-D-arabinofuranosyl)luracil

1-(β-D-Arabinofuranosyl)-5-prop-1-ynyluracil (J. Med. Chem. 26(5) 661-666 [1983]) (0.31g 1.1mmol) was dissolved in dry pyridine (5ml) and to it was added isobutyric anhydride (0.6ml 0.57g, 3.63mmol) and the whole was stirred at room temperature for 6 hours then stored in the refrigerator overnight. The reaction mixture was evaporated to dryness and residual pyridine co-evaporated with portions of ethanol. The residue was chromatographed on silica gel using 2.5% $MeOH/CH_2Cl_2$ and the final product isolated following trituration with ether/hexane.
Yield : 0.13g (24%)
Mpt : 112-113°C

| | | | |
|---|---|---|---|
| Anal. Calc. | C-58.54, | H-6.504, | N-5.69% |
| Found | C-58.80, | H-6.426, | N-5.623% |

Example 46

5-Prop-1-ynyl-1-(2,3,5-tri-O-propionyl-β-D-arabinofuranosyl)uracil

1-(β-D-Arabinofuranosyl)-5-prop-1-ynyluracil (J. Med. Chem. 26(5) 661-666 [1983]) (0.31g 1.1mmol) was dissolved in dry pyridine (5ml) and propionic anhydride (0.5ml) (0.62g, 3.96mmol) was added and the whole was stirred at room temperature for 6 hours. The solvent was evaporated and the residual pyridine co-evaporated with portions of ethanol. The remaining oil was chromatographed on a silica gel column eluting with 4% $MeOH/CH_2Cl_2$, the product triturated with ether/40.60 pet ether to give a white solid which was dissolved in $CH_2Cl_2$, the solution washed with $NaHCO_3$ solution, dried and evaporated to dryness. The residue was then triturated with ether/hexane filtered to give the title compound as a white solid.
Yield : 0.28g (57%)
Mpt : 97-100°C

| | | | |
|---|---|---|---|
| Anal. Calc. | C-56.00, | H-5.778, | N-6.222% |
| Found | C-56.06, | H-5.709, | N-6.094% |

Example 47

5-Prop-1-ynyl-1-(2,3,5-tri-O-valeryl-β-D-arabinofuranosyl)uracil

1-(β-D-Arabinofuranosyl)-5-prop-1-ynyluracil (J. Med. Chem. 26(5) 661-666 [1983]) (0.31g 1.1mmol) was dissolved in dry pyridine (5ml) and the solution stirred at 0°C. To this was added valeryl chloride (0.43ml) (3.63mmol, 0.44g) and the whole was stirred at room temperature for 6 hours then stored in the refrigerator overnight. The mixture was poured over ice and the aqueous solution was extracted with $CH_2Cl_2$ (3x25ml), the organic layer was dried over $Na_2SO_4$ and evaporated to dryness. The residue was chromatographed on silica gel eluting with 3% MeOH/$CH_2Cl_2$ to give the title compound as an oil.

Yield : 0.08g (14%)

Example 48

5-Prop-1-ynyl-1-(2,3,5-tri-O-acetyl-β-D-arabinofuranosyl)uracil

1-(β-D-Arabinofuranosyl)-5-iodouracil prepared according to EP 0272065 (1g, 2.7mmol)was dissolved in dry pyridine (10ml) and to the solution was added acetic anhydride (0.84ml) (8.9mmol) and the mixture was stirred at room temperature for 2 hours. A further 0.2ml of acetic anhydride was added and stirred for 1 hour. The solvent was evaporated, residual pyridine coevaporated with portions of EtOH, the residue triturated with ethanol, the mixture filtered and dried. A suspension of the product (1.16g 2.3mmol), CuI (35mg), $(Ph_3P)_2PdCl_2$ (35mg) in dry triethylamine (95ml) was stirred under dry $N_2$ for 15 minutes. Propyne gas was bubbled through the suspension for 15 minutes and the mixture was stirred at 50°C under dry $N_2$. The solvent was evaporated to dryness, the residue dissolved in $CH_2Cl_2$ (50ml), the solution washed with 2% aqueous EDTA solution (2x25ml) water (25ml) dried over $Na_2SO_4$, filtered and evaporated to dryness. The residue was redissolved in hot $CH_2Cl_2$ (5ml), 30ml of ethanol was added and the mixture was left to crystallise. The solid was filtered washed with ethanol then further recrystallised from ethanol to give an analytically pure sample of the title compound.

Yield : 0.38g (37%)

Mpt : 150-151°C

| | | | |
|---|---|---|---|
| Anal. Calc. | C-52.94, | H-4.902, | N-6.863% |
| Found | C-52.86, | H-4.827, | N-6.784% |

Example A

Ophthalmic Solution

| | |
|---|---|
| Active ingredient | 0.5 |
| Sodium chloride, analytical grade | 0.9 g |
| Thiomersal | 0.001 g |
| Purified Water to | 100 ml |
| pH adhusted to | 7.5 |

Example B: Tablet Formulations

The following formulations A, B and C are prepared by wet granulation of the ingredients with a solution of povidone, followed by addition of magnesium stearate and compression.

Formulation A

| | mg/tablet | mg/tablet |
|---|---|---|
| (a) Active ingredient | 250 | 250 |
| (b) Lactose B.P. | 210 | 26 |
| (c) Povidone B.P. | 15 | 9 |
| (d) Sodium Starch Glycollate | 20 | 12 |
| (e) Magnesium Stearate | 5 | 3 |
| | 500 | 300 |

Formulation B

| | mg/tablet | mg/tablet |
|---|---|---|
| (a) Active ingredient | 250 | 250 |
| (b) Lactose | 150 | - |
| (c) Avicel PH 101 | 60 | 26 |
| (d) Povidone B.P. | 15 | 9 |
| (e) Sodium Starch Glycollate | 20 | 12 |
| (f) Magnesium Stearate | 5 | 3 |
| | 500 | 300 |

Formulation C

| | mg/tablet |
|---|---|
| Active ingredient | 100 |
| Lactose | 200 |
| Starch | 50 |
| Povidone | 5 |
| Magnesium stearate | 4 |
| | 359 |

The following formulations, D and E, are prepared by direct compression of the admixed ingredients. The lactose used in formulation E is of the direct compression type.

Formulation D

| | mg/tablet |
|---|---|
| Active Ingredient | 250 |
| Pregelatinised Starch NF15 | 150 |
| | 400 |

Formulation E

| | mg/tablet |
|---|---|
| Active Ingredient | 250 |
| Lactose | 150 |
| Avicel | 100 |
| | 500 |

Formulation F (Controlled Release Formulation)

The formulation is prepared by wet granulation of the ingredients (below) with a solution of povidone followed by the addition of magnesium stearate and compression.

| | mg/tablet |
|---|---|
| (a) Active Ingredient | 500 |
| (b) Hydroxypropylmethylcellulose (Methocel K4M Premium) | 112 |
| (c) Lactose B.P. | 53 |
| (d) Povidone B.P.C. | 28 |
| (e) Magnesium Stearate | 7 |
| | 700 |

Drug release takes place over a period of about 6-8 hours and was complete after 12 hours.

Example C: Capsule Formulations

Formulation A

A capsule formulation is prepared by admixing the ingredients of Formulation D in Example B above and filling into a two-part hard gelatin capsule. Formulation B (infra) is prepared in a similar manner.

Formulation B

| | mg/capsule |
|---|---|
| (a) Active ingredient | 250 |
| (b) Lactose B.P. | 143 |
| (c) Sodium Starch Glycollate | 25 |
| (d) Magnesium Stearate | 2 |
| | 420 |

Formulation C

| | mg/capsule |
|---|---|
| (a) Active ingredient | 250 |
| (b) Macrogol 4000 BP | 350 |
| | 600 |

Capsules are prepared by melting the Macrogol 4000 BP, dispersing the active ingredient in the melt and filling the melt into a two-part hard gelatin capsule.

Formulation D

| | mg/capsule |
|---|---|
| Active ingredient | 250 |
| Lecithin | 100 |
| Arachis Oil | 100 |
| | 450 |

Capsules are prepared by dispersing the active ingredient in the lecithin and arachis oil and filling the dispersion into soft, elastic gelatin capsules.

Formulation E (Controlled Release Capsule)

The following controlled release capsule formulation is prepared by extruding ingredients a, b, and c using an extruder, followed by spheronisation of the extrudate and drying. The dried pellets are then coated with release- controlling membrane (d) and filled into a two-piece, hard gelatin capsule.

| | mg/capsule |
|---|---|
| (a) Active Ingredient | 250 |
| (b) Microcrystalline Cellulose | 125 |
| (c) Lactose BP | 125 |
| (d) Ethyl Cellulose | 13 |
| | 513 |

Example D: Injectable Formulation

| | |
|---|---|
| Active ingredient | 0.200 g |
| Sterile, pyrogen free phosphate buffer (pH 7.0) to | 10 ml |

The active ingredient is dissolved in most of the phosphate buffer (35-40°C) then made up to volume and filtered through a sterile micropore filter into a sterile 10ml amber glass vial (type 1) and sealed with sterile closures and overseals.

Example E: Intramuscular injection

| | | |
|---|---|---|
| Active Ingredient | | 0.20 g |
| Benzyl Alcohol | | 0.10 g |
| Glycofurol 75 | | 1.45 g |
| Water for Injection | q.s. to | 3.00 ml |

The active ingredient is dissolved in the glycofurol. The benzyl alcohol is then added and dissolved, and water added to 3 ml. The mixture is then filtered through a sterile micropore filter and sealed in sterile 3 ml glass vials (type 1).

Example F: Syrup Suspension

| | | |
|---|---|---|
| Active ingredient | | 0.2500 g |
| Sorbitol Solution | | 1.5000 g |
| Glycerol | | 2.0000 g |
| Dispersible Cellulose | | 0.0750 g |
| Sodium Benzoate | | 0.0050 g |
| Flavour, Peach 17.42.3169 | | 0.0125 ml |
| Purified Water | q.s. to | 5.0000 ml |

The sodium benzoate is dissolved in a portion of the purified water and the sorbitol solution added. The active ingredient is added and dispersed. In the glycerol is dispersed the thickener (dispersible cellulose). The two dispersions are mixed and made up to the required volume with the purified water.

Example G: Suppository

| | mg/suppository |
|---|---|
| Active Ingredient (63μm) | 250 |
| Hard Fat, BP (Witepsol H15 - Dynamit NoBel) | 1770 |
| | 2020 |

* The active ingredient is used as a powder wherein at least 90% of the particles are of 63μm diameter or less.

One-fifth of the Witepsol H15 is melted in a steam-jacketed pan at 45°C maximum. The active ingredient is sifted through a 200μm sieve and added to the molten base with mixing, using a silverson fitted with a cutting head, until a smooth dispersion is achieved. Maintaining the mixture at 45°C, the remaining Witepsol H15 is added to the suspension and stirred to ensure a homogenous mix. The entire suspension is passed through a 250μm stainless steel screen and, with continuous stirring, is allowed to cool to 40°C. At a temperature of 38°C to 40°C 2.02g of the mixture is filled into suitable plastic moulds. The suppositories are allowed to cool to room temperature.

Example H: Pessaries

| | mg/pessary |
|---|---|
| Active ingredient 63μm | 250 |
| Anhydrous Dextrose | 380 |
| Potato Starch | 363 |
| Magnesium Stearate | 7 |
| | 1000 |

The above ingredients are mixed directly and pessaries prepared by direct compression of the resulting mixture.

Example I: Topical formulation

| Cream | | |
|---|---|---|
| Active compound | | 5.00g |
| Glycerol | | 2.00g |
| Cetostearyl alcohol | | 6.75g |
| Sodium lauryl sulphate | | 0.75g |
| White soft paraffin | | 12.50g |
| Liquid paraffin | | 5.00g |
| Chlorocresol | | 0.10g |
| Purified water | to | 100.00g |

Dissolve the active compound in a mixture of purified water and glycerol and heat to 70°C. Heat the remaining ingredients together at 70°C. Add the two parts together and emulsify. Cool and fill into containers.

Antiviral and Toxicity Testing

Varicella Zoster Virus (VZV) is assayed in monolayers of MRC5 cells (human embryonic lung) in multiwell trays. Activity of compounds is determined in the plaque reduction assay, in which a cell monolayer is infected with a suspension of VZV. A range of concentrations of the compound to be tested (of known molarity) is then incorporated into the cell monolayer. Plaque numbers of each concentration are expressed as percentage of the control and a dose-response curve is drawn. From this curve the 50% inhibitory concentration ($IC_{50}$) is estimated.

Human cytomegalovirus (HCMV) is assayed in MRC5 cells or Detroit 532 cells (human foreskin fibroblasts) by a similar method of that for VZV with the addition of an agarose overlay into which the range of concentrations of the compound of known molarity, may be incorporated.

An assay is performed in which virus-producing cells (P3HR-1) are exposed to drug for 14 days after which the EBV genome copies per cell are determined by EBV specific c-RNA-DNA hybridization. Epstein Barr virus is assayed by the methods of Nomoyama & Pagano disclosed in Nature: New Biology Vol. 233, pg. 103-4 1971. The $IC_{50}$ value given in the results is the concentration required to inhibit the EBV genome No/cell by 50%

Cell toxicity is assessed in cell growth inhibition assay. Subconfluent cultures of Vero cells grown on 96-well microtiter dishes are exposed to different dilutions of drug, and cell viability determined daily on replicate cultures using uptake of a tetrazolium dye (MTT). The concentration required for a 50% inhibition of cell vaibility at 96 hours is termed $CCID_{50}$.

Results

| Example | $ID_{50}$(μm) VZV | $CCID_{50}$ |
|---|---|---|
| 20 | <1.0 | >500 |

Determination of Oral Bioavailability

Long Evans Rats were administered the compound to be tested by gavage at a dose of 50 mg/kg. The urine was collected for 24 and 48 hours post-dose, ultrafiltered, and analysed by reverse-phase high-pressure liquid chromatography. The oral bioavailability of the compound was expressed as the percent of the dose excreted in the urine as the parent unesterified compound.

| Compound | | Urinary Recovery (% of dose) as parent compound |
|---|---|---|
| Example | 1 | 40.35 |
| | 12 | 51.18 |
| | 20 | 35.90 |

**Claims**

1. A compound of formula (I):

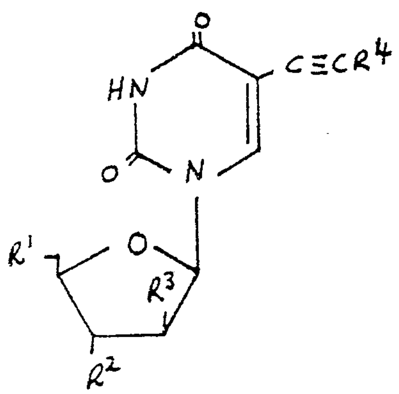

(I)

wherein $R^1$ represents a hydroxy group, an amino acid ester group or a carboxylic acid ester group in which the non-carbonyl moiety of the ester grouping is selected from straight or branched chain $C_{1-6}$ hydroxyalkyl or $C_{1-6}$ alkyl, $C_{3-7}$ cycloalkyl, heterocycloalkyl, heterocycloalkenyl, $C_{1-6}$ alkoxy, $C_{1-6}$ alkoxyalkyl, $C_{1-6}$ carboxyalkyl, $C_{1-6}$ aminoalkyl, carbamoylalkyl, aralkyl, aryloxyalkyl, aryl optionally substituted by halogen, $C_{1-6}$ alkyl or $C_{1-6}$ alkoxy, or a mono-, di- or tri-phosphate ester, or an ether group; $R^2$ represents a hydroxy group, an amino acid or a carboxylic acid ester group as defined for $R^1$, a monophosphate ester, or an ether group; $R^3$ represents a hydrogen atom, a hydroxy group, or an ester group, or an ether group as defined for $R^1$; $R^4$ represents a hydrogen atom or a methyl group; or a pharmaceutically acceptable salt thereof provided that at least one of $R^1$, $R^2$ and $R^3$ represents an ester or an ether group and provided that when $R^4$ is hydrogen, $R^3$ is not hydrogen or $R^1$, $R^2$ and $R^3$ do not all represent acetyl groups; or provided that when $R^4$ is a methyl group and $R^3$ is hydrogen, $R^1$ and $R^2$ do not both represent toluoyl groups or do not both represent acetyl groups; or provided that when $R^4$ is methyl, $R^1$, $R^2$ and $R^3$ do not all represent toluoyl groups or do not all represent acetyl groups or when $R^2$ and $R^3$ represent hydroxy groups, $R^1$ does not represent a morpholinocarbonyl, dimethylcarbonyl, carboxypropionyl, t-butoxycarbonyl group or when $R^1$ and $R^2$ represent hydroxy groups, $R^3$ does not represent a dimethylaminocarbonyl group.

2. A compound according to claim 1 wherein $R^4$ represents a methyl group.

3. A compound according to claims 1 or 2 wherein $R^2$ and $R^3$ represent hydroxy groups.

4. A compound according to any of the preceding claims wherein $R^1$ represents a carboxylic acid ester grouping.
5. A compound according to claim 4 wherein the non-carbonyl moiety of the carboxylic acid ester grouping is a straight or branched chain $C_{1-6}$alkyl group.
6. A compound according to claim 4 wherein the non-carbonyl moiety of the carboxylic acid ester grouping is a straight or branched chain $C_{1-6}$alkoxy group.
7. A compound according to claim 4 wherein the non-carbonyl moiety of the carboxylic acid ester grouping is a $C_{3-7}$ cycloalkyl group.
8. A compound according to any of claims 1-3 wherein $R^1$ represents an amino acid ester group.
9. A compound of formula (I) selected from:1-(5-0-Isobutyryl-β-D-arabinofuranosyl)-5-prop-1-ynyluracil;
1-(5-0-Cyclohexylcarbonyl-β-D-arabinofuranosyl)-5-prop-1-ynyluracil;
1-(5-0-(3-Methylbutyryl)-β-D-arabinofuranosyl)-5-prop-1-ynyluracil;
1-(5-0-Ethoxycarbonyl-β-D-arabinofuranosyl)-5-prop-1-ynyluracil;
5-Prop-1-ynyl-1-(3-0-trimethylacetyl-β-D-arabinofuranosyl)uracil;
1-(5-0-Butyryl-β-D-arabinofuranosyl)-5-prop-1-ynyluracil;
1-(5-0-(2-Ethylbutyryl)-β-D-arabinofuranosyl)-5-prop-1-ynyluracil;
5-Prop-1-ynyl-1-(5-0-L-valyl-β-D-arabinofuranosyl)-uracil.
10. A compound of formula (I)

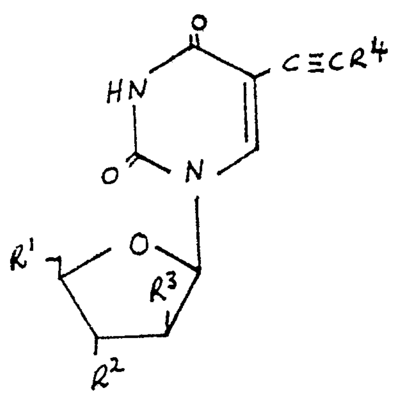

(I)

wherein $R^1$ represents a hydroxy group, an amino acid ester group or a carboxylic acid ester group in which the non-carbonyl moiety of the ester grouping is selected from straight or branched chain $C_{1-6}$ hydroxyalkyl or $C_{1-6}$ alkyl, $C_{3-7}$ cycloalkyl, heterocycloalkyl, heterocycloalkenyl, $C_{1-6}$ alkoxy, $C_{1-6}$ alkoxyalkyl, $C_{1-6}$ carboxyalkyl, $C_{1-6}$ aminoalkyl, carbamoylalkyl, aralkyl, aryloxyalkyl, aryl optionally substituted by halogen, $C_{1-6}$ alkyl or $C_{1-6}$ alkoxy, or a mono-, di- or tri-phosphate ester, or an ether group; $R^2$ represents a hydroxy group, an amino acid or a carboxylic acid ester group as defined for $R^1$, a monophosphate ester, or an ether group; $R^3$ represents a hydrogen atom, a hydroxy group, or an ester group, or an ether group as defined for $R^1$; $R^4$ represents a hydrogen atom or a methyl group; or a pharmaceutically acceptable salt thereof provided that at least one of $R^1$, $R^2$ and $R^3$ represents an ester or an ether group and provided that when $R^4$ is hydrogen, $R^3$ is not hydrogen or $R^1$, $R^2$ and $R^3$ do not all represent acetyl groups; or provided that when $R^4$ is a methyl group and $R^3$ is hydrogen, $R^1$ and $R^2$ do not both represent toluoyl groups or do not both represent acetyl groups; or provided that when $R^4$ is methyl, $R^1$, $R^2$ and $R^3$ do not all represent toluoyl groups or do not all represent acetyl groups or when $R^2$ and $R^3$ represent hydroxy groups, $R^1$ does not represent a morpholinocarbonyl, dimethylcarbonyl, carboxypropionyl, t-butoxycarbonyl group or when $R^1$ and $R^2$ represent hydroxy groups, $R^3$ does not represent a dimethylaminocarbonyl group for use in medical therapy.
11. A compound of formula (I) according to claim 10 for use in the treatment or prophylaxis of a VZV infection.
12. A compound of formula (I) according to claim 10 for use in the treatment or prophylaxis of an EBV infection.
13. A process for the preparation of a compound of formula (I) as defined in claim 1 comprising:
A. reacting a compound of formula (II)

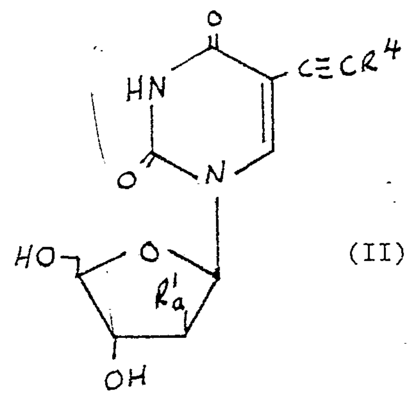

(II)

wherein $R^4$ is as hereinbefore defined, and $R^1_a$ is a hydroxy group or an appropriate hydroxy-protecting group with a compound serving to provide the appropriate ester grouping(s) at the 2,3 and/or 5-position of the sugar; or

B. reacting a compound of formula (III)

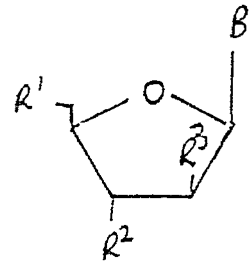

(III)

wherein $R^1$, $R^2$ and $R^3$ are as hereinbefore defined and B is a purine or pyrimidine base, with a 5-alkynyl uracil base; or

C. reacting a compound of formula (IV)

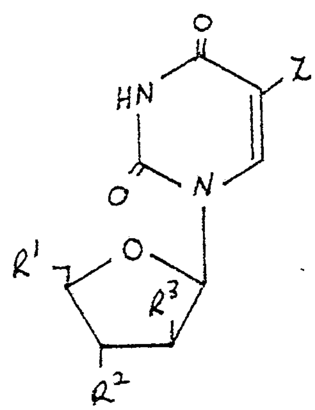

(IV)

wherein Z is a leaving group and $R^1$, $R^2$ and $R^3$ are as hereinbefore defined with a compound serving to provide the appropriate alkynyl group, optionally thereafter or simultaneously therewith, performing either or both of the following, in any desired order:

i) removing any protecting groups;

ii) where the resulting compound is a compound of formula (I), converting it into a pharmaceutically acceptable salt thereof or, where the resulting compound is pharmaceutically acceptable salt, converting it into a different pharmaceutically acceptable salt or a compound of formula (I).

14. A pharmaceutical formulation comprising as active ingredient a compound of formula (I) as defined in claim 1 and a pharmaceutically acceptable carrier therefor.

**Claims for the following Contracting States: ES, GR.**

1. A process for the preparation of a compound of formula (I)

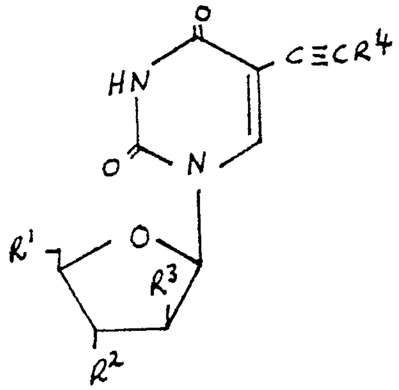

wherein $R^1$ represents a hydroxy group, an amino acid ester group or a carboxylic acid ester group in which the non-carbonyl moiety of the ester grouping is selected from straight or branched chain $C_{1-6}$ hydroxyalkyl or $C_{1-6}$ alkyl, $C_{3-7}$ cycloalkyl, heterocycloalkyl, heterocycloalkenyl, $C_{1-6}$ alkoxy, $C_{1-6}$ alkoxyalkyl, $C_{1-6}$ carboxyalkyl, $C_{1-6}$ aminoalkyl, carbamoylalkyl, aralkyl, aryloxyalkyl, aryl optionally substituted by halogen, $C_{1-6}$ alkyl or $C_{1-6}$ alkoxy, or a mono-, di- or tri-phosphate ester, or an ether group; $R^2$ represents a hydroxy group, an amino acid or a carboxylic acid ester group as defined for $R^1$, a monophosphate ester, or an ether group; $R^3$ represents a hydrogen atom, a hydroxy group, or an ester group, or an ether group as defined for $R^1$; $R^4$ represents a hydrogen atom or a methyl group; or a pharmaceutically acceptable salt thereof provided that at least one of $R^1$, $R^2$ and $R^3$ represents an ester or an ether group and provided that when $R^4$ is hydrogen, $R^3$ is not hydrogen or $R^1$, $R^2$ and $R^3$ do not all represent acetyl groups; or provided that when $R^4$ is a methyl group and $R^3$ is hydrogen, $R^1$ and $R^2$ do not both represent toluoyl groups or do not both represent acetyl groups; or provided that when $R^4$ is methyl, $R^1$, $R^2$ and $R^3$ do not all represent toluoyl groups or do not all represent acetyl groups or when $R^2$ and $R^3$ represent hydroxy groups, $R^1$ does not represent a morpholinocarbonyl, dimethylcarbonyl, carboxypropionyl, t-butoxycarbonyl group or when $R^1$ and $R^2$ represent hydroxy groups, $R^3$ does not represent a dimethylaminocarbonyl group, comprising:

A. reacting a compound of formula (II)

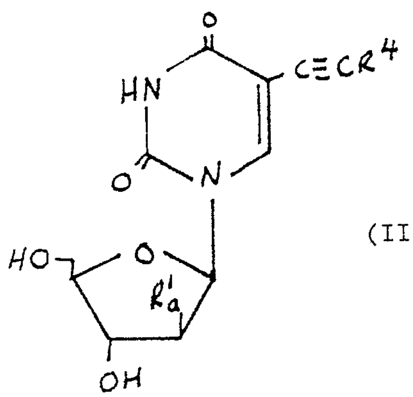

(II)

wherein $R^4$ is as hereinbefore defined, and $R^1_a$ is a hydroxy group or an appropriate hydroxy-protecting group with a compound serving to provide the appropriate ester grouping(s) at the 2,3 and/or 5-position of the sugar; or

B. reacting a compound of formula (III)

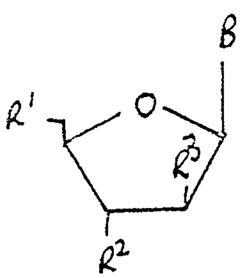

(III)

wherein $R^1$, $R^2$ and $R^3$ are as hereinbefore defined and B is a purine or pyrimidine base, with a 5-alkynyl uracil base; or

C. reacting a compound of formula (IV)

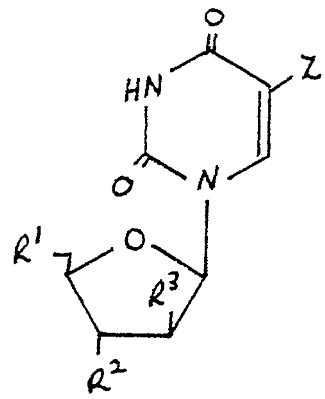

(IV)

wherein Z is a leaving group and $R^1$, $R^2$ and $R^3$ are as hereinbefore defined with a compound serving to provide the appropriate alkynyl group,
optionally thereafter or simultaneously therewith, performing either or both of the following, in any desired order:
i) removing any protecting groups;
ii) where the resulting compound is a compound of formula (I), converting it into a pharmaceutically acceptable salt thereof or, where the resulting compound is pharmaceutically acceptable salt, converting it into a different pharmaceutically acceptable salt or a compound of formula (I).

2. A process according to claim 1 wherein $R^4$ represents a methyl group.

3. A process according to claim 1 wherein $R^2$ and $R^3$ represent hydroxy groups.

4. A process according to claim 1 wherein $R^1$ represents a carboxylic acid ester grouping.

5. A process according to claim 4 wherein the non-carbonyl moiety of the carboxylic acid ester grouping is a straight or branched chain $C_{1-6}$ alkyl group.

6. A process according to claim 4 wherein the non-carbonyl moiety of the carboxylic acid ester grouping is a straight or branched chain $C_{1-6}$ alkoxy group.

7. A process according to claim 4 wherein the non-carbonyl moiety of the carboxylic acid ester grouping is a $C_{3-7}$ cycloalkyl group.

8. A process according to claim 1 wherein $R^1$ represents an amino acid group.

9. A process for the preparation of a pharmaceutical formulation comprising the preparation of a compound of formula (I)

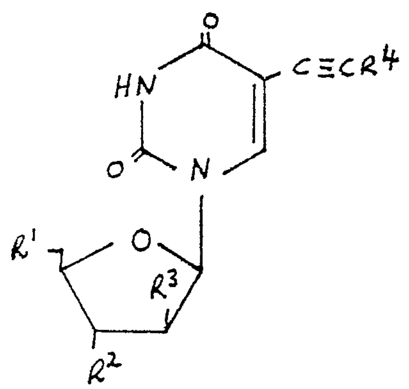

(I)

wherein $R^1$ represents a hydroxy group, an amino acid ester group or a carboxylic acid ester group in which the non-carbonyl moiety of the ester grouping is selected from straight or branched chain $C_{1-6}$ hydroxyalkyl or $C_{1-6}$ alkyl, $C_{3-7}$ cycloalkyl, heterocycloalkyl, heterocycloalkenyl, $C_{1-6}$ alkoxy, $C_{1-6}$ alkoxyalkyl, $C_{1-6}$ carboxyalkyl, $C_{1-6}$ aminoalkyl, carbamoylalkyl, aralkyl, aryloxyalkyl, aryl optionally substituted by halogen, $C_{1-6}$ alkyl or $C_{1-6}$ alkoxy, or a mono-, di- or tri-phosphate ester, or an ether group; $R^2$ represents a hydroxy group, an amino acid or a carboxylic acid ester group as defined for $R^1$, a monophosphate ester, or an ether group; $R^3$ represents a hydrogen atom, a hydroxy group, or an ester group, or an ether group as defined for $R^1$; $R^4$ represents a hydrogen atom or a methyl group; or a pharmaceutically acceptable salt thereof provided that at least one of $R^1$, $R^2$ and $R^3$ represents an ester or an ether group and provided that when $R^4$ is hydrogen, $R^3$ is not hydrogen or $R^1$, $R^2$ and $R^3$ do not all represent acetyl groups; or provided that when $R^4$ is a methyl group and $R^3$ is hydrogen, $R^1$ and $R^2$ do not both represent toluoyl groups or do not both represent acetyl groups; or provided that when $R^4$ is methyl, $R^1$, $R^2$ and $R^3$ do not all represent toluoyl groups or do not all represent acetyl groups or when $R^2$ and $R^3$ represent hydroxy groups, $R^1$ does not represent a morpholinocarbonyl, dimethylcarbonyl, carboxypropionyl, t-butoxycarbonyl group or when $R^1$ and $R^2$ represent hydroxy groups, $R^3$ does not represent a dimethylaminocarbonyl group, comprising:

A. reacting a compound of formula (II)

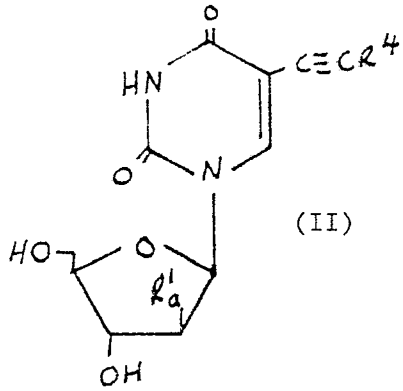

(II)

wherein $R^4$ is as hereinbefore defined, and $R^1_a$ is a hydroxy group or an appropriate hydroxy-protecting group with a compound serving to provide the appropriate ester grouping(s) at the 2,3 and/or 5-position of the sugar; or

B. reacting a compound of formula (III)

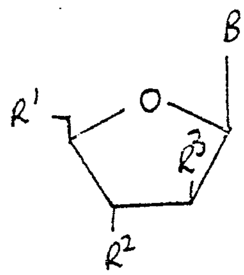

(III)

wherein $R^1$, $R^2$ and $R^3$ are as hereinbefore defined and B is a purine or pyrimidine base, with a 5-alkynyl uracil base; or

C. reacting a compound of formula (IV)

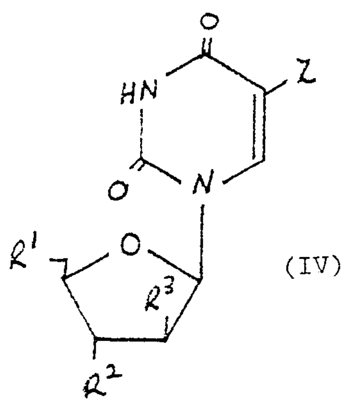

(IV)

wherein Z is a leaving group and $R^1$, $R^2$ and $R^3$ are as hereinbefore defined with a compound serving to provide the appropriate alkynyl group, optionally thereafter or simultaneously therewith, performing either or both of the following, in any desired order:

i) removing any protecting groups;

ii) where the resulting compound is a compound of formula (I), converting it into a pharmaceutically acceptable salt thereof or, where the resulting compound is pharmaceutically acceptable salt, converting it into a different pharmaceutically acceptable salt or a compound of formula (I).

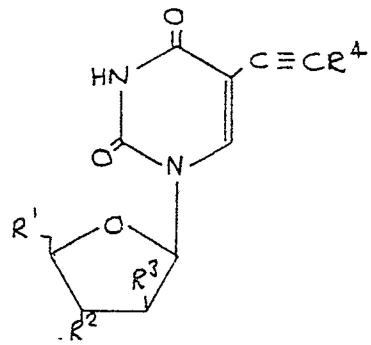

(I)

and then combining said compound with a pharmaceutically acceptable carrier therefor.